# EUROPEAN PATENT SPECIFICATION

(11) **EP 0 337 713 B1**
(45) Date of publication and mention of the grant of the patent: **18.10.1995**
(21) Application number: 89303537.8
(22) Date of filing: 11.04.1989
(51) Int. Cl.: C07D 473/32, C07D 473/34, C07D 405/04, A61K 31/52, A61K 31/505

(54) **2-Substituted-4-Substituted-1,3-Dioxolanes, Synthesis and use thereof**
2- und 4-substituierte 1,3-Dioxolane, deren Synthese und Verwendung
1,3-Dioxolanes substitués en 2 et 4, leur synthèse et leur utilisation

(30) Priority: 11.04.1988 US 179615
(43) Date of publication of application: 18.10.1989
(73) Proprietor: BIOCHEM PHARMA INC., Laval, Quebec H7V 4A7 (CA)
(72) Inventor: Belleau, Bernard, Westmount Quebec H3Y 2R3 (CA); Dixit, Dilop, Montreal Quebec H2X 2E2 (CA); Nguyen-Ba, Nghe, Brossard Quebec JAZ 1G6 (CA)
(74) Representative: Ritter, Stephen David

(56) References cited:
- EP-A- 0 206 497

## Description

### FIELD OF THE INVENTION

The present invention relates to novel 2-substituted-4-substituted-1,3 dioxolanes which are useful as antiviral agents.

### PRIOR ART

Retroviral infections are a serious cause of disease and among others the acquired immunodeficiency syndrome (AIDS) is an immunosuppressive disease associated with life-threatening opportunistic infections and high susceptibility to unusual neoplasms (Kaposi sarcoma for instance). The human immunodeficiency virus (HIV) has been recognized as the etiologic agent of AIDS and compounds having an inhibitory effect against HIV multiplication have been actively sought.

One product which has been proposed for the treatment of AIDS is the 3′-azido-2′,3′-dideoxythymidine commonly referred to as AZT. The activity of this compound was disclosed by MITSUYA et al. in Proc. Natl. Acad. Sci., U.S.A. 1985, 82, 7096. The compound has the structure: 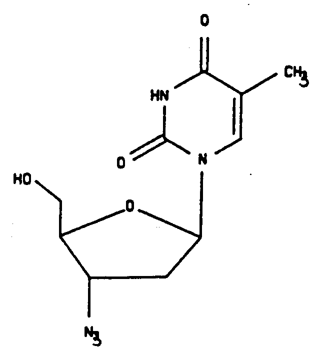
This compound is useful in protecting AIDS carriers against the cytopathogenic effect of the human immunodeficiency virus (HIV) which is the etiologic agent of AIDS.

Mitsuya et al. have also disclosed in Proc. Natl. Acad. Sci., U.S.A. 1986, 86, 1911 a group of 2′,3′-dideoxynucleosides which appear to possess potent protective activity against HIV-induced cytopathogenicity. A typical such compound is the 2′,3′-dideoxycytidine of the formula: 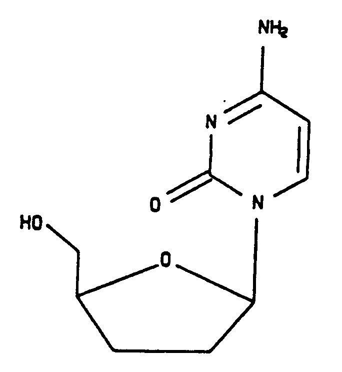
Balzarini et al. in Biochem. Biophys. Res. Comm. 1986, 140, 735 disclose that the unsaturated analog of the 2′,3′-dideoxycytidine also possesses antiretroviral effectiveness. This unsaturated analog has the formula: 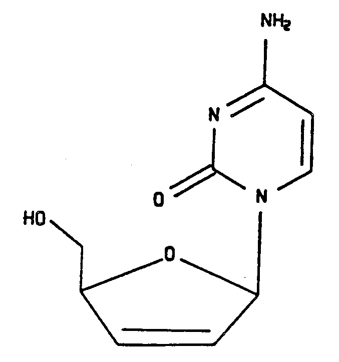
Baba et al. in Biochem. Biophys. Res. Comm. 1987, 142, 128 have described the 2′,3′-unsaturated analog of the 2′,3′-dideoxythymidine which is a potent selective inhibitor of HIV replication and which corresponds to the formula: 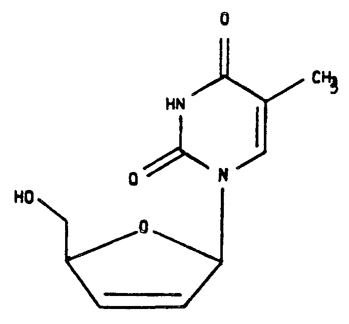
Analogues of the 3′-azido-2′,3′-dideoxythymidine are the 3′-azido-2′,3′-dideoxyuridine of the formula: 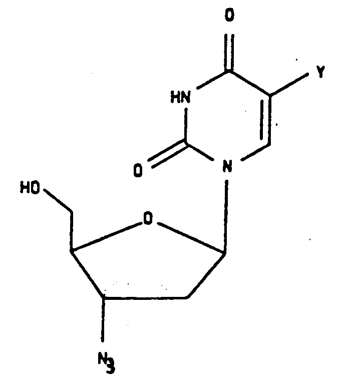
where y is bromine or iodine. These have been disclosed as having an inhibitory activity against Moloney murine leukemia by T.S. Lin et al., in J. Med. Chem. 1987, 30, 440.

Finally, the 3′fluoro analogues of the 2′,3′-dideoxycytidine and of the 2′,3′-dideoxythymidine have been disclosed by Herdewijn et al. in J. Med. Chem. 1987, 30, 1270 as having potent antiretroviral activity (anti-HIV). These analogues correspond to the formulae: 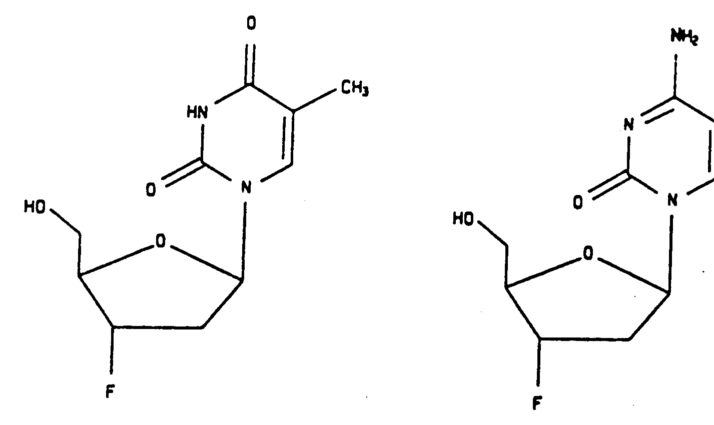
The most potent anti-HIV compounds thus far reported are 2′,3′-dideoxynucleosides, more particularly, 2′,3′-dideoxycytidine (ddCyd) and 3′-azido-2′,3′-dideoxythymidine (AzddThd or AZT). These compounds are also active against other kinds of retroviruses (such as the Moloney murine leukemia virus). It is because of the increasing incidence and the life-threatening characteristics of AIDS that efforts are being expended to discover and develop new non-toxic and potent inhibitors of HIV and blockers of its infectivity.

It is therefore an object of the present invention to provide effective anti-HIV compounds of low toxicity and a synthesis of such new compounds that is readily feasible.

### SUMMARY OF THE INVENTION

In accordance with the present invention there are provided novel 2-substituted-4-substituted-1,3-dioxolanes which are particularly useful as antiviral agents.

More specifically, the novel 2-substituted- 4-substituted-1,3-dioxolane derivatives of the present invention correspond to the following formula (L): 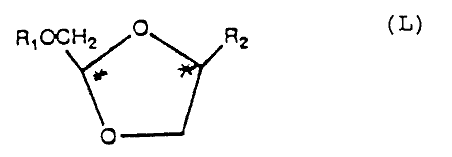
wherein R₁ is selected from the group consisting of hydrogen, an acyl group having 1 to 16 carbon atoms, benzoyl and a benzoyl substituted in any position by at least one halogen, C₁ - C₃ alkyl, C₁ - C₃ alkoxy, nitro and trifluromethyl groups; R₂ is a heterocyclic radical selected from: 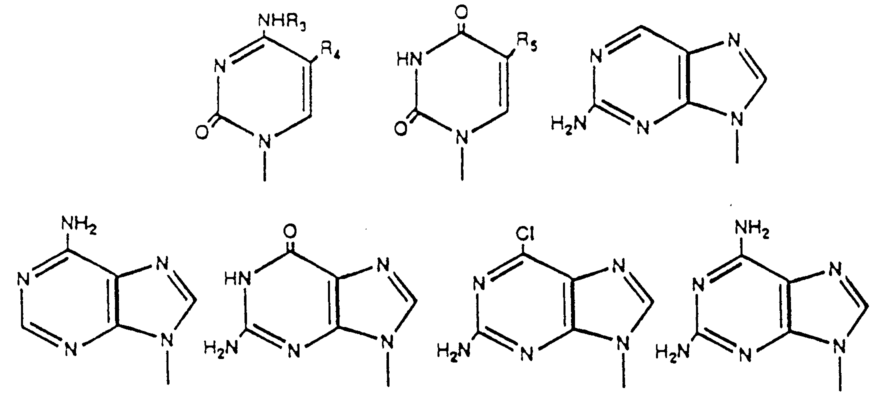
wherein R₃ and R₄ are independently selected from the group consisting of hydrogen and C₁ - C₃ alkyl and R₅ is selected from the group consisting of C₁ - C₃ alkyl and halogen.

Also within the scope of the present invention are the 2,4-disubstituted-1,3-dioxolanes of Formula (L) wherein R₂ could be any nucleoside base analog, those base analogs being known by those of skill in the art of nucleoside chemistry.

There are two asymmetric carbons (asterisks) in the disubstituted 1,3-dioxolane molecule which provide for two racemic forms (±) and therefore four optical isomers. These racemates differ in the relative configurations of the 2- and 4-substituents which can either assume the cis- and trans-configurations. The use of a graphic representation of the 2,4-disubstituted-1,3-dioxolanes of Formula (L) is meant to include the dl racemic mixture as well as the separate d and l isomers thereof.

Also in accordance with the present invention, there is provided a pharmaceutical composition for administration to persons infected with the AIDS virus or other infectious agent which comprises a therapeutically effective amount of the 2,4-disubstituted dioxolane of Formula (L) or pharmaceutically acceptable salt in association with a pharmaceutically acceptable excipient. The amount of active ingredient which is contained in a single dosage form will vary depending upon the host treated as well as the frequency and the mode of administration.

Also within the scope of the present invention is a method for treating AIDS-infected persons which comprises administering to said persons a therapeutically effective amount of 2,4-disubstituted-1,3-dioxolane of Formula (L). Also within the scope of the present invention is any combination of a 2,4-disubstituted-1,3-dioxolane of Formula (L) with another drug where such combination is therapeutically more advantageous than either drug given above.

### DETAILED DESCRIPTION OF THE INVENTION

The compounds of the invention are prepared starting from glycerol and 2-halo- (or an equivalent leaving group such as aryl- or alkyl-sulfonyloxy) acetaldehyde preferably in the form of an acetal derivative according to the reported procedure of E.G. Hallonquist and H. Hibbert, Can. J. Res. 1933, 7, 129. For the purpose of this disclosure, the term acyl is an alkanoyl radical of 2 to 16 carbon atoms, e.g., acetyl, propionyl, isobutyryl, myristoyl, etc. The compounds of the instant invention are prepared by a total synthesis comprising a few steps. The synthesis is practical and is commercially feasible. The process for preparing one specific compound of the present invention is outlined in the following Flowsheet 1:

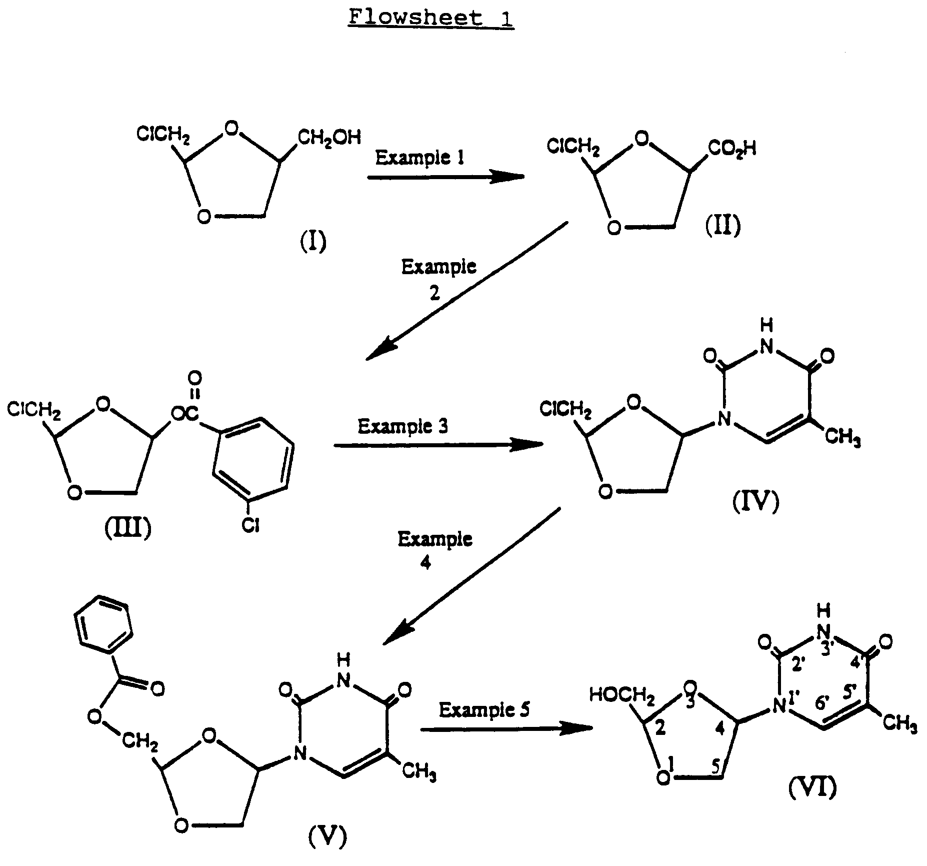

The various steps involved in the synthesis illustrated in Flowsheet 1 may be briefly described as follows:
Step 1 The primary alcohol function of the starting dioxolane I is treated with an oxidizing reagent such as chromic acid (which may be complexed with pyridine) in a compatible organic solvent to give the corresponding dioxolane carboxylic acid II.
Step 2 The acid II is converted to a mixed anhydride using an alkyl chloroformate and submitted to a Bayer-Villiger oxidation with an organic peracid such as m-chloroperbenzoic acid to yield the corresponding aroyloxydioxolane III.
Step 3 Intermediate III is then reacted with thymine previously silylated with hexamethyldisilazane in a compatible solvent and the reaction catalyzed by a Lewis acid or preferably by trimethylsilyltriflate to give the thymin-1′-yl dioxolane IV.
Step 4 The chlorine atom of IV is displaced by reaction with a benzoic acid salt in a compatible solvent such as dimethylformamide to give intermediate V.
Step 5 The benzoate ester function is then hydrolyzed under basic conditions to yield the desired end-product VI.

An alternate process for preparing further specific compounds of the present invention is illustrated in Flowsheet II: 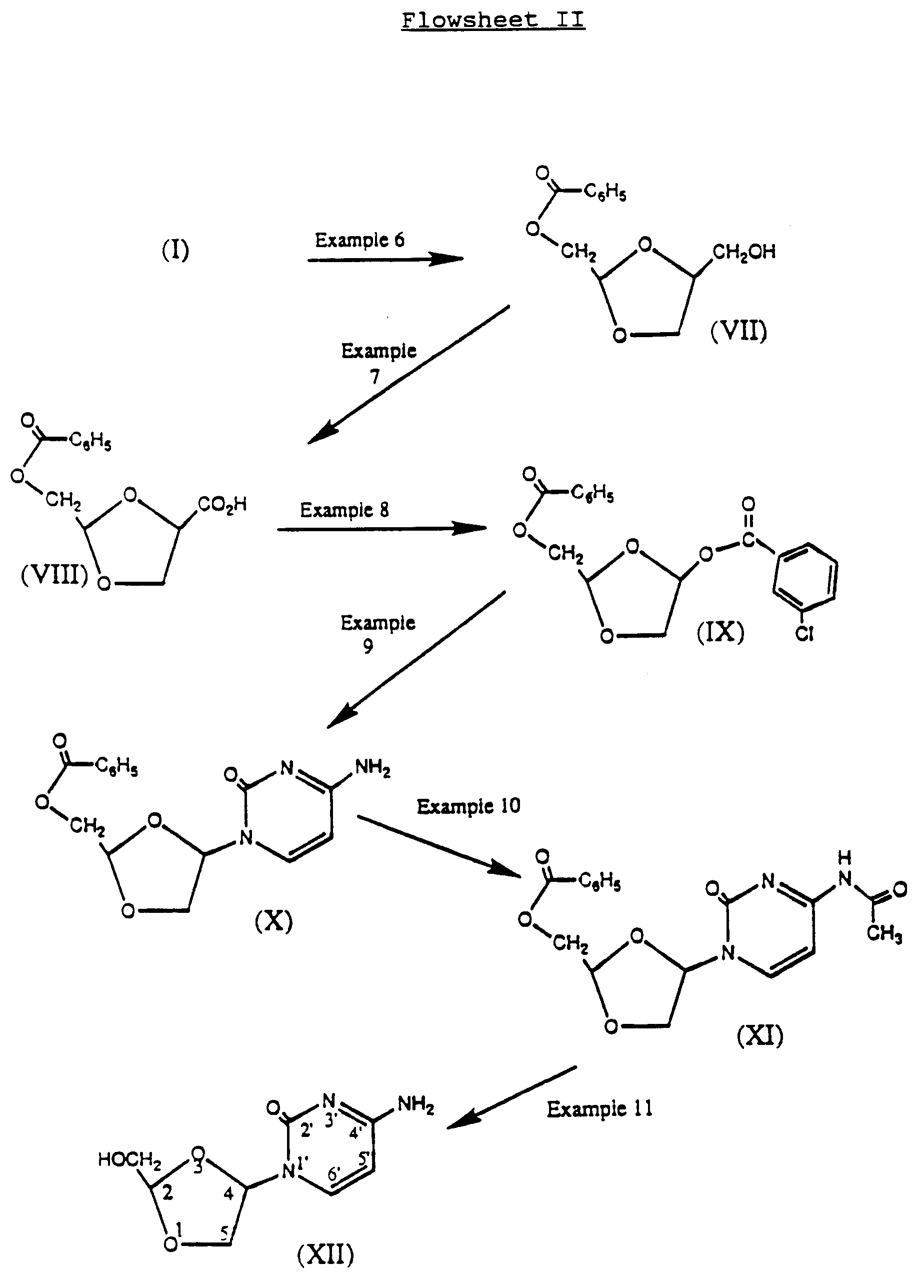 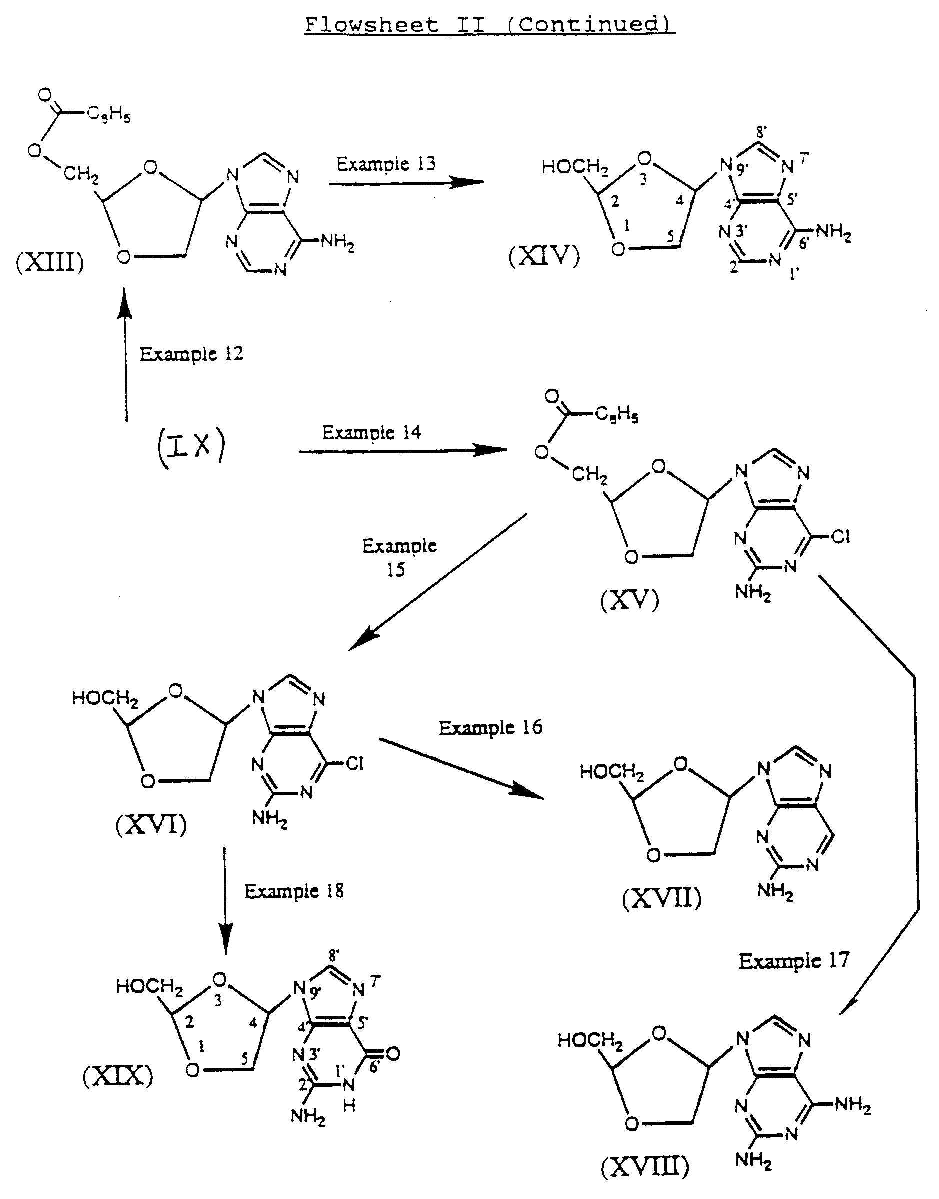
The various steps involved in the synthesis illustrated in Flowsheet II may be briefly described as follows:
Step 1 The chlorine atom of starting dioxolane I is displaced by a benzoic (or acetic) acid salt in a solvent such a dimethylformamide to yield the diol monoester VII.
Step 2 The hydroxymethyl group of VII is oxidized with a suitable reagent such as chromic acid (which may be complexed with pyridine) in a compatible organic solvent to give the dioxolane carboxylic acid VIII.
Step 3 The acid VIII is then submitted to Bayer-Villiger oxidation by the procedure outlined in Step 2 (Flowsheet 1) above to give the corresponding aroyloxy-dioxolane IX.
Step 4 The key intermediate IX is reacted with cytosine previously silylated under the reaction conditions outlined in Step 3 (Flowsheet 1) to give the cytosin-1'-yl dioxolane X.
Step 5 The amine function of X is acylated with acetic anhydride in pyridine to give XI which provides for easier separation of isomers.
Step 6 The ester and acetyl functions of XI are hydrolyzed under basic conditions to yield the desired end-product XII.
Step 7 (IX to XIII) Key intermediate IX is reacted with adenine by the procedure outlined above in Step 3 (Flowsheet 1) to give XIII.
Step 8 (XIII to XIV) The ester function of XIII is hydrolyzed under basic conditions to yield the desired end-product XIV.
Step 9 (IX to XV) Intermediate IX is reacted with 2-amino-6-chloropurine under the conditions outlined in Step 3 (Flowsheet 1) to give compound XV.
Step 10 (XV to XVI) The preceding intermediate is hydrolyzed under basic conditions to yield the desired end-product XVI.
Step 11 (XVI to XVII) The chlorine atom of XVI is removed by catalytic hydrogenation over Pd/C to give the 2′-amino-purin-9′-yl dioxolane XVII.
Step 12 The above intermediate XV is reacted with excess ammonia under pressure whereupon the 2′,6′-diamino-purin-9′-yl dioxolane XVIII is generated.
Step 13 Compound XVI is submitted to boiling sodium hydroxide to give the desired end-product guanin-9′-yl dioxolane XIX.

### ANTIVIRAL ACTIVITY

All of the compounds of the preferred embodiments are novel and some are valuable for their properties as non-toxic inhibitors of the primary replication of HIV-1 in previously uninfected T-lymphocytes over a prolonged period of time.

In particular, the compounds having the formula XII possess desirable properties, i.e., antagonism of HIV infectivity towards T-lymphocytes in the absence of cytotoxicity.

In vitro testing was conducted on the compounds to determine their inhibitory properties. Table 1 represents the results of a typical experiment. The figures reported are the micromolar concentrations in the incubation media which effect the ability of T-lymphocyte H-9 cells to be infected by HIV-1 following the protocol of H. Mitsuya and S. Broder, Proc. Natl. Acad. Sci. U.S.A. 1986, 83, 1911-1915; the level of infection being measured by the level of reverse transcriptase activity (RTA) as assayed in the usual manner with tritiated thymidine triphosphate (TTP). As a control drug, 2′,3′-dideoxy-3′-azido-thymidine (AZT) was used and the RTA measured in the incubation medium after 8, 12 and 26 days of exposure to the inhibitor. The values in Table 1 reflect the total number of virus particles in the incubation medium.

**Table 1**

| Example of the effects of prototype compounds trans-XII, cis-XIV and AZT on ability of H-9 cells to be infected by HIV-1. | | | | | |
|---|---|---|---|---|---|
| Expt.# | Inhibitor | Conc.- | RTA activity (cpm) after: | | |
| | | | 8 days | 12 days | 26 days |
| 1 | none | - | 198,612 | 327,570 | 239,019 |
| | trans-XII | 10µM | 4,608 | 83,462 | 312,478 |
| | trans-XII | 50µM | 1,319 | 758 | 1,732 |
| | AZT | 20µM | 633 | 419 | 821 |
| 2 | none | - | 64,769 | 119,580 | 227,471 |
| | cis-XIV | 20µM | 2,618 | 130,563 | 210,583 |
| | cis-XIV | 50µM | 1,132 | 39,752 | 231,609 |
| | AZT | 20µM | 587 | 1,316 | 679 |

It is apparent from the table that prototype compound trans-XII exhibits potent inhibitory activity. Other analogues displayed variable degrees of antiviral activity. Accordingly, as it is the rule for certain analogues of nucleosides, trans-XII and selected analogues are expected to demonstrate in vivo activity as inhibitors of retroviruses. Such compounds may also be used in combination with other antiviral agents at reduced doses, thus lowering their toxicity potential.

### TOXICITY

In contrast to the results obtained with AZT or with other di-deoxynucleosides analogues, in vitro toxicity experiments showed that the compound trans-XII is non toxic even at concentration as high as 200µM. In spite of the AZT activity, its serious bone marrow toxicity limits its therapeutic usefulness. It is then highly desirable to provide with new active antiviral agents which would be devoided of toxic side effects.

### EXAMPLES

### Example 1. Preparation of 2-chloromethyl-1,3-dioxolane-4-carboxylic acid (II).

Starting material I (40 g; prepared according to E.G. Hallonquist and H. Hibbert, Can. Res. J. 1933, 7, 129) was treated with pyridinium dichromate (PDC; 345 g) in dimethyl formamide (DMF; 690ml) at 0° according to the procedure of E.J. Corey and G. Schmidt, Tetrahedron Lett., 1979, 399 and product II obtained as a crude mixture of cis- and trans-isomers (20 g) was identified by its ¹H NMR spectrum [200 MHz, CDCl₃; tetramethyl silane (TMS) as internal reference]
δ(ppm): 3.6-3.8 (m,2H;CH₂Cl);
4.1-4.5 (m,2H;C₅H₂);
4.72-4.797 (gg,1H;C₄-H);
5.29-5.46 (tt,1H;C₂-H).
The product was used as such in the next step.

### Example 2. Preparation of 2-chloromethyl-4-m.chlorobenzoyloxy-1,3-dioxolane (III).

The preceding product II (5.26 g) was treated in CH₂Cl₂ at -20° with 3.6 ml of ethyl chloroformate in the presence of 4.5 mg of triethylamine. To the solution was added 8.85 g of m.chloroperbenzoic acid at room temperature according to the procedure of D.H.R. Barton, I.H. Coates and P.G. Sammes, J. Chem. Soc., Perkin 1, 1973, 599 to give III as a mixture of cis- and trans-isomers. These were separated and purified by flash chromatography on silica gel using a mixture of hexane and ethyl acetate as the eluent. The isomers were identified by their ¹H NMR spectra (recorded as in example 1): trans-isomer of III: δ(ppm):
3.66 (q,2H;CH₂-Cl);
4.36 (qq,2H;C₅-H₂);
5.57 (t,1H;C₂-H);
6.7 (q,1H;C₄-H);
7.39-8.0 (m,4H;aromatic H);
cis-isomer of III: δ(ppm):
3.66 (q,2H;CH₂-Cl);
4.24 (qq,2H;C₅-H₂);
5.43 (t,1H;C₂-H);
6.63 (q,1H;C₄-H);
7.42-8.04 (m,4H;aromatic H).

### Example 3. Preparation of 2-chloromethyl-4-(thymin-1′-yl)-1,3-dioxolane (IV).

Reaction of the preceding compound with thymine was carried out according to the procedure of D.S. Wise and L.B. Townsend, in Nucleic Acid Chemistry, Eds. L.B. Townsend and R.S. Tipson, John Wiley & Sons, Inc., New York, 1978, Part 1, pp. 413-419. The product was a mixture of cis- and trans-isomers of IV (37.3 mg from 131 mg of III) which had the following ¹H NMR characteristics (obtained as in example 1):
δ(ppm): 1.93 (d,3H;5′-CH₃);
3.64 and 3.85 (dd,2H;CH₂Cl);
4.17-4.46 (m,2H;C₅-H₂);
5.26 and 5.72 (tt,1H;C₂-H);
6.6 and 6.66 (qq,1H;C₄-H);
7.40 and 7.49 (dd,1H;C_{6'}-H);
U.V.: (CH₃OH)λmax.264 nm.

### Example 4. Preparation of 2-acetoxymethyl-4-(thymin-1′-yl)-1,3-dioxolane (V).

The preceding compound IV (35 mg) was reacted with anhydrous potassium acetate (70 mg) in boiling DMF (3 ml) for 4h to give after conventional workup a cis- and trans-mixture of V (25 mg). These isomers were purified and separated by flash chromatography on silica using a mixture of hexane and ethyl acetate as the eluent. Their ¹H NMR spectra were as follows:
trans-isomer of V: δ(ppm):
1.94 (d,3H;C_{5'}-CH₃);
2.12 (s,3H;CH₃CO₂-);
4.05-4.43 (m,4H;C₂-CH₂-O₂CCH₃ and C₅-H₂);
5.65 (t,1H;C₂-H);
6.31 (q,1H;C₄-H);
7.14 (d,1H;C_{6'}-H);
8.18 (m,1H;N_{3'}-H).
cis-isomer of V: δ(ppm):
1.97 (d,3H;C_{5'}CH₃);
2.14 (s,3H;CH₃CO-O);
4.13-4.49 (m,4H;2-CH₂OCOCH₃ and C₅H₂);
5.19 (t,1H;C₂-H);
6.40 (q,1H;C₄H);
7.43 (d,1H;C_{6'}-H);
8.12 (m,1H;N_{3'}-H).
U.V.: (CH₃OH) λmax.264 nm.

### Example 5. Preparation of 2-hydroxymethyl-4-(thymin-1'-yl)-1,3-dioxolane (VI).

The preceding trans- and cis-isomers of V (10 mg) were respectively treated with a catalytic amount of potassium carbonate in methanol (5 ml) at room temperature for 5-6 h and the mixture worked up in the usual manner and the respective products purified by flash chromatography on silica gel using a mixture of ethyl acetate and methanol as the eluent The ¹H NMR spectrum of the pure trans-isomer of VI was as follows (in CD₃COCD₃ as solvent);
trans-VI: δ(ppm):
1.87 (d,3H;C_{5'}-CH₃);
3.61 (q,2H;C₂-CH₂OH);
4.30 (qq,2H;C₅-H₂);
5.56 (t,1H;C₂-H);
6.31 (q,1H;C₄-H);
7.41 (d,1H;C_{6'}H).
U.V.: (CH₃OH) λmax.265 nm.
cis-isomer of VI (in CD₃COCD₃): δ(ppm):
1.82 (d,3H;C_{5'}-CH₃);
3.82 (q,2H;C₂CH₂OH);
4.24 (qq,2H;C₅-H₂);
5.02 (t,1H;C₂-H);
6.34 (q,1H;C₄-H);
7.81 (d,1H;C_{6'}-H).
U.V.: (CH₃OH) λmax.264nm.

### Example 6. Preparation of 2-benzoyloxymethyl-4-hydroxymethyl-1,3-dioxolane (VII).

Starting material I (41.6 g) was treated with potassium benzoate (65.56 g) in boiling dimethyl formamide containing 100 mg of 18-crown-6 for 24 h after which time the mixture was worked up in the usual manner and the product (51.02 g) characterized by its ¹H NMR spectrum (CDCl₃;TMS):
δ(ppm): 3.5-4.8 (m,H;C₅-H₂;C₂-CH₂OCOC₆H₅,C₄-CH₂OH and C₂-H);
5.05 and 5.16 (tt,1H;C₄-H);
7.27-8.10 (m,5H; aromatic H).
Similar results were obtained using potassium acetate instead of potassium benzoate.

### Example 7. Preparation of 2-benzoyloxymethyl-1,3-dioxolane-4-carboxylic acid (VIII).

The preceding compound VII (51.02 g) was treated at 0° with pyridinium dichromate (282.5 g) in dimethyl formamide (565 ml) and the mixture worked up in the usual manner to give 35 g of crude VIII which was used as such in the next example.

### Example 8.

A 10 g portion of crude VIII was treated with 6.03 ml of ethyl chloroformate in the presence of 8.6 ml of triethylamine followed by the addition of 16.81 g of m.chloroperbenzoic acid exactly as described in example 2 for the case of the preparation of intermediate III. The isomers of product IX thus obtained were purified by flash chromatography on silica gel using a mixture of hexane and ethyl acetate as the eluent. They were characterized by their ¹H NMR spectra (CDCl₃):
trans-isomer of IX: δ(ppm):
4.29 (qq,2H;C₅-H₂);
4.49 (d,2H;C₂-CH₂OCOC₆H₅);
5.66 (t,1H;C₂-H);
6.70 (q,1H;C₄-H);
7.27-8.10 (m,9H; aromatic).
cis-isomer of IX: δ(ppm):
4.27 (qq,2H;C₅-H₂);
4.51 (d,2H;C₂-CHOCOC₆H₅);
5.51 (t,1H;C₂-H);
6.59 (d,1H;C₄-H);
7.26-8.09 (m,9H; aromatic).

### Example 9. Preparation of 2-benzoyloxymethyl-4-(cytosin-1'-yl)-1,3-dioxolane (X).

Following the procedure described by T. Ueda and S.I. Watanabe, Chem. Pharm. Bull. (Japan), 1985, 33, 3689-3695 and by G. Gosselin, M.C. Bergogne, J. DeRudder, E. DeClercq and J.L. Imbach, J. Med. Chem., 1987, 30, 982-991, cytosine (139 mg) and either isomer of the proceding compound IX (363 mg) yielded a mixture of cis- and trans-isomers (390 mg) X which were used as such in the following step.

### Example 10.

Treatment of cis- and trans-X with excess acetic anhydride in pyridine at room temperature yielded after work up in the conventional manner, a mixture of the cis- and trans-isomers of XI which were separated and purified by flash chromatography on silica gel using a mixture of hexane and ethyl acetate as the eluent. They were characterized by their ¹H NMR spectra (CDCl₃):
trans-isomer of XI: δ(ppm):
2.15 (s,3H;C_{4'}-NH-COCH₃);
4.16 and 4.46 (m,4H;C₅-H₂ and C₂-CH₂OCOC₆-H₅);
5.96 (t,1H;C₂-H);
6.24 (q,1H;C₄-H);
7.55-8.09 (m,5H;aromatic);
8.15 (d,1H;C_{6'}-H).
cis-isomer of XI: δ(ppm):
2.15 (s,3H;C_{4'}-NH-COCH₃);
4.26 and 4.56 (m,4H;C₅-H₂ and C₂-CH₂OCOC₆-H₅);
5.35 (t,1H;C₄-H);
6.25 (q,1H;C₄-H);
7.18 (d,1H;C_{5'}-H);
7.58-8.04 (m,5H; aromatic);
8.17 (d,1H;C_{6'}-H).

### Example 11. Preparation of cis- and trans-2-hydroxymethyl-4-(cytosin-1'-yl)-1,3-dioxolane (XII).

Each of the preceding isomers of XI (25 mg) was treated with potassium carbonate (20 mg) in methanol at room temperature for several hours and the mixtures worked in the usual manner to yield each isomer of XII which were purified by chromatography on silica gel using a mixture of ethyl acetate and methanol as eluent. They were crystallized from methanol and characterized by their respective ¹H NMR spectra (D₃CCOCD₃):
trans-isomer of XII: δ(ppm):
3.62 (q,2H;C₂-CH₂OH);
m.p.179-180° 4.21 (qq,2H;C₅-H₂);
5.50 (t,1H;C₂-H);
5.93 (d,1H;C_{5'}-H,J=7.5Hz);
6.18 (q,1H;C₄-H);
7.66 (d,1H;C_{6'}-H,J=7.5Hz).
U.V.: (CH₃OH) λmax.271 nm.
cis-isomer of XII: δ(ppm):
3.82 and 4.15 (m,4H;C₅-H₂ and C₂-CH₂OH);
m:p.173-174° 5.04 (t,1H;C₂-H);
5.83 (d,1H;C_{5'}-H);
6.23 (q,1H;C₄-H);
8.05 (d,1H;C_{6'}H).
U.V.: (CH₃OH) λmax.270nm.

### Example 12. Preparation of 2-benzoyloxymethyl-4-(adenin-9'-yl)-1,3-dioxolane (XIII).

Following the same procedure as in example 9, adenosine (135 mg) was coupled with either isomer of intermediate IX (545 mg) in dimethylformamide at 120° in the presence of trimethylsilyl triflate (0.45 ml) and the mixture worked up in the usual manner to yield a mixture of cis- and trans-isomers of XIII (540 mg) which were purified and separated by chromatography on silica gel using a mixture of hexane and ethyl acetate as the eluent. They were characterized by their respective ¹H NMR spectra (CDCl₃):
trans-isomer of XIII: δ(ppm):
4.5 and 4.59 (m,4H;C₅-H₂ and C₂-CH₂OCOC₆H₅);
6.00 (t,1H;C₂-H);
6.65 (q,1H;C₄-H);
6.75 (m,2H;C_{6'}H₂);
7.68-8.21 (m,5H;aromatic);
8.36 (s,1H;C_{2'}-H);
8.37 (s,1H;C_{8'}-H).
cis-isomer of XIII: δ(ppm):
4.62 (d,2H;C₂-CH₂OCOC₆H₅);
4.65 (qq,2H;C₅-H₂);
5.52 (t,1H;C₂-H);
6.59 (q,1H;C₄-H);
6.85 (m,2H;C_{6'}-NH₂);
6.96-7.71 (m,5H;aromatic);
7.66 (d,2H;C_{2'}-H and C_{8'}-H).

### Example 13. Preparation of 2-hydroxymethyl-4-(adenin-9'-yl)-1,3-dioxolane (XIV).

Each isomer of the preceding compound XIII was treated with potassium carbonate in methanol at room temperature by the same procedure described in example 5 and each product purified by column chromatography on silica gel using a mixture of ethyl acetate and methanol as the eluent. The isomers were further purified by crystallization from methanol and characterized by their ¹H NMR spectra (CD₃SOCD₃):
trans-isomer of XIV: δ(ppm):
3.50 (d,2H;C₂-CH₂OH);
4.70 (m,2H;C₅-H₂);
5.52 (t,1H;C₂-H);
6.44 (q,1H;C₄-H);
8.18 (s,1H;C_{2'}-H);
8.31 (s,1H;C_{8'}-H).
U.V.: (CH₃OH) λmax.269 nm.
cis-isomer of XIV: δ(ppm):
4.63 (d,2H;C₂-CH₂OH);
4.29 (qq,2H;C₅-H₂);
5.08 (t,1H;C₂-H);
6.43 (q,1H;C₄-H);
8.18 (s,1H;C_{2'}-H);
8.36 (s,1H;C_{8'}-H).
U.V.: (CH₃OH) λmax.269 nm.

### Example 14. Preparation of 2-benzoyloxymethyl-4-(2'-amino-6'-chloro-purin-9'-yl)-1,3-dioxolane (XV).

A solution of 2-amino-6-chloropurine (600 mg; 3.54 mmol) in 20 ml of hexamethyldisilazane (HMDS) containing 0.5 ml of trimethylsilyl chloride (TMS-Cl) was heated under reflux for 3 h after which time the mixture was evaporated to dryness in vacuo. The residue was dissolved in 75 ml of dichloroethane containing 910 mg of compound IX and 0.6 ml of trimethylsilyl triflate (TMS-T_{f}) added. After refluxing under argon for 4 h, the mixture was collected, 2 g of solid NaHCO₃ added followed by 50 ml of saturated aqueous NaHCO₃. The organic layer was collected and after work-up in the usual manner, crude XV was obtained as an oil which was purified and separated into its isomer by chromatography on silica gel using hexane-ethyl acetate (3:7) as the eluent to give 230 mg of pure trans- and 250 mg or pure cis-isomer as colorless foams. They were characterized by their ¹H NMR spectra (CDCl₃):
trans-isomer of XV (R :0.40; hexane-EtOAc 3:7):
δ(ppm): 4.45-4.52 (m,4H;C₅-H₂,C₂-CH₂OCOC₆H₅);
5.16 (b,2H;C_{2'}-NH₂);
5.83 (t,1H;C₂-H,J=3.8 Hz);
6.39 (dd,1H;C₄-H);
7.41-7.58 (m,3H;aromatic);
7.92 (s,1H;C_{8'}-H);
8.06 (d,2H;aromatic,J=7Hz).
U.V.: (CH₃OH) λmax. 312 nm.
cis-isomer of XV (R :0.26; hexane-EtOAc 3:7):
δ(ppm): 4.25-4.33 (dd,1H;C₅-H,J=5.43 Hz);
4.59-4.64 (m,3H;C₅-H and C₂-CH₂-OCOC₆H₅);
5.17 (b,2H;C_{2'}-NH₂);
5.42 (t,1H;C₂-H,J=3.50 Hz);
6.33-6.53 (dd,1H;C₄-H);
7.38-7.57 (m,3H;aromatic);
7.93-7.98 (d,2H;aromatic);
8.00 (s,1H;C_{8'}-H).
U.V.: (CH₃OH) λmax.312 nm.

### Example 15. Preparation of trans- and cis-2-hydroxymethyl-4-(2'-amino-6'-chloro-purin-9'-yl)-1,3-dioxolane (XVI).

The preceding trans-isomer of XV (180 mg) was dissolved in 30 ml of methanol, the solution cooled to 0° and dry ammonia bubbled through for 15 min. After stirring at room temperature for 15 h, the solvent was removed in vacuo and the residue crystallized from ether. After recrystallization from ethanol-ether, 98 mg of pure trans-XVI, m.p. 155-156°, was obtained (R_{f}: 0.23, EtOAc). It was characterized by ¹H NMR (DMSO-d₆):
trans-XVI: δ(ppm): 3.44-3.49 (m,2H;C₂-CH₂OH);
4.37-4.45 (m,2H;C₅-H₂);
5.01 (t,1H;C₅-CH₂OH,J=6.2Hz);
5.46 (t,1H;C₂-H,J=3.6 Hz);
6.27-6.32 (dd,1H;C₄-H,J=4,1 Hz);
7.00 (b,2H;C_{2'}-NH₂);
8.26 (s,1H;C_{8'}-H).
U.V.: (CH₃OH) λmax.247 and 308 nm.
The cis-isomer of XVI was obtained in similar yield from the cis-isomer of XV by the same preceding procedure. After recrystallization from ethanol-ether, the pure product had m.p. 145-147° (R_{f}:0.24, EtOAc). It was characterized by ¹H NMR (DMSO-d₆):
cis-XVI: δ(ppm): 3.54-3.59 (m,2H;C₂-CH₂OH);
4.12-4.19 (dd,1H;C₅-H,J=5.3 Hz and 9.8 Hz);
4.48-4.53 (d,1H;C₅-H,J=9.8 Hz);
5.01 (t,1H;C₂-H,J=2.8 Hz);
5.09 (t,1H;C₂-CH₂-OH,J=6.0 Hz);
6.24 (d,1H;C₄-H,J=5.1 Hz);
6.96 (b,2H;C_{2'}-NH₂);
8.23 (s,1H;C_{8'}-H).
U.V.: (CH₃OH) λmax.247 and 308 nm.

### Example 16. Preparation of trans-and cis-2-hydroxymethyl-4-(2'-amino-purin-9'-yl)-1,3-dioxolane (XVII).

The preceding trans-isomer of XVI (50 mg) was submitted to hydrogenation conditions under 345 kPa [50 (psi)] of hydrogen over 10% Pd/C (30 mg) in 30 ml of ethanol containing 0.5 ml of triethylamine. After 3 h of shaking, the mixture was worked up in the usual manner to yield a solid which was recrystallized from ethanol-ether to give 36 mg of pure trans-XVII, m.p. 153-155°, R_{f}:0.25 (EtOAc: MeOH 85:15). It was characterized by ¹H NMR (DMSO-d₆):
trans-XVII: δ(ppm): 3.44-3.49 (m,2H;C₂-CH₂OH);
4.38-4.44 (m,2H;C₅-H₂);
4.99 (t,1H;C₂-CH₂-OH,J=6.1 Hz);
5.45 (t,1H;C₂-H,J=3.6 Hz);
6.29-6.34 (dd,1H;C₄-H);
6.59 (b,2H;C_{2'}-NH₂);
8.19 (s,1H;C_{8'}-H);
8.59 (s,1H;C_{6'}-H).
The cis-isomer of XVII was obtained in similar yield from the cis-isomer of XVI by the same preceding procedure. After recrystallisation from ethanol- ether, the pure product had m.p. 145-148°, R_{f}: 0.25 (EtOAc:MeOH 85:15). It was characterized by ¹H NMR (DMSO-d₆):
cis-XVII: δ(ppm): 3.55-3.60 (dd,2H;C₂-CH₂H,J=2.10
and 6.1 Hz);
4.14-4.22 (dd,1H;C₅-H,J=5.4 and 9.7 Hz);
4.47-4.53 (dd,1H;C₅-H,J=1.38 and 9.7 Hz);
5.02 (t,1H;C₂-H,J=3 Hz);
5.11 (t,1H;C₂-CH₂OH,J=7.2 Hz);
6.58 (b,2H;C₂-NH₂);
8.19 (s,1H;C_{8'}-H);
8.57 (s,1H;C_{6'}-H).
U.V.: (CH₃OH) λmax. 255, 308 nm.

### Example 17. Preparation of trans- and cis-2-hydroxymethyl-4-(2',6'-diamino-purin-9'-yl)-1,3-dioxolane (XVIII):

The above compound trans-XV (200 mg) was dissolved in 30 ml of methanol saturated at 0° with dry ammonia and the solution heated in a steel bomb to 105-110° for 16h. The solution was evaporated to dryness and the residue purified by chromatography on silica gel using chloroform-methanol 4:1 as the eluent to give 101 mg of product which was recrystallized from methanol-ether to yield pure trans-XVIII, m.p. 165-168°, R_{f}:0.30(CHCl₃:CH₃OH 4:1). It was characterized by ¹H NMR (DMSO-d₆):
trans-XVIII: δ(ppm):3.43-3.48 (m,2H;C₂-CH₂OH);
4.34-4.49 (m,2H;C₅-H₂);
4.97 (t,1H;C₂-CH₂OH);
5.42 (t,1H;C₂-H);
5.82 (b,2H;C_{2'}-or C_{6'}-NH₂);
6.18-6.23 (dd,1H;C₄-H);
6.72 (b,2H;C_{2'}- or C_{6'}-NH₂);
7.84 (s,1H;C_{8'}-H).
U.V.: (CH₃OH) λmax.255,280 nm.
The cis-isomer of XVIII was obtained by the same preceding procedure from compound cis-XV. After recrystallization from methanol-ether, pure cis-XVIII, m.p. 180-182°, R_{f}:0.32(CHCl₃:CH₃OH 4:1) was obtained in a similar yield. It was characterized by ¹H NMR (DMSO-d₆):
cis-XVIII: δ(ppm): 3.56-3.58 (d,2H;C₂-CH₂OH,J=4.2Hz);
4.11-4.19 (dd,1H;C₅-H,J=4.5 and 9.7Hz);
4.38-4.44 (dd,1H;C₅-H,J=1.6 and 11.2Hz);
5.00 (t,1H;C₂-H,J=3.1 Hz);
5.91 (b,2H;C_{2'}- or C_{6'}-NH₂);
6.15-6.19 (dd,1H;C₄-H);
6.84 (b,2H;C_{2'}- or C_{6'}-NH₂);
7.86 (s,1H;C_{8'}-H).
U.V.: (CH₃OH) λmax.254,279 nm.

### Example 18. Preparation of cis- and trans-2-hydroxymethyl-4-(guanosin-9'-yl)-1,3-dioxolane (XIX).

The above cis-XVI (40 mg) was dissolved in a mixture of 15 ml of methanol, 2 ml of water and 2 g of sodium hydroxide and the solution heated under reflux for 5 h after which time it was diluted with 100 ml of water and excess pyridinium sulfonate resin added. The slurry was filtered, the resin washed with water and the combined aqueous filtrates evaporated to dryness in vacuo to leave a residue which was taken up in 50% aqueous methanol. The solution was treated with activated charcoal, filtered and the filtrate evaporated to dryness in vacuo to give a solid residue that was recrystallized from ethanol-water to yield pure cis-XIX (27 mg) m.p. > 250° decomp., R_{f}:0.23 (CHCl₃:CH₃OH 7:3). It was characterized by ¹H NMR (DMSO-d₆):
cis-XIX: δ(ppm): 3.55 (m,2H;C₂CH₂OH);
4.10-4.17 (dd,1H;C₅-H,J=5.6 and 9.8 Hz);
4.37-4.42 (dd,1H;C₅-H,J=1.4 and 9.6 Hz);
4.98 (t,1H;C₂-H,J=3.2 Hz);
5.15 (b,1H;C₂-CH₂OH);
6.10-6.13 (dd,1H;C₄-H,J=2.4 and 5.3 Hz);
6.66 (b,2H;C_{2'}-NH₂);
7.78 (s,1H;C_{8'}-H);
11.02 (b,1H;N_{1'}-H). U.V.: (CH₃OH) λmax.252, 270 nm (shoulder).
The isomer trans-XIX was obtained in similar yield from the above trans-XVI by the same preceding procedure. After recrystallization from ethanol-water, pure trans-XIX, m.p. > 260°(dec.), R_{f}:0.23 (CHCl₃:CH₃OH 7:3) was obtained and characterized by ¹H NMR (DMSO-d₆):
trans-XIX: δ(ppm): 3.42-3.47 (m,2H;C₂-CH₂OH);
4.34 (d,2H;C₅-H₂,J=4.8Hz);
4.99 (t,1H;C₂-CH₂OH);
5.40 (t,1H;C₂-H,J=3.5 Hz);
6.15-6.20 (t,1H;C₄-H,J=4.8 Hz);
6.49 (b,2H;C_{2'}-NH₂);
7.83 (s,1H;C_{8'}-H);
10.64 (b,1H;N_{1'}-H).
U.V.: (CH₃OH) λmax.252, 270 (shoulder)

## Claims (Claims for the following Contracting State(s): AT, BE, CH, DE, FR, GB, IT, LI, LU, NL, SE)

1. A 1,3-dioxolane derivative of the general formula (L), the geometric and optical isomers thereof, and mixtures of these isomers: 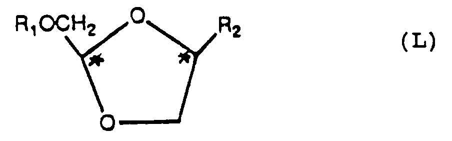 wherein:
R₁ is selected from the group consisting of hydrogen, an acyl group having 1 to 16 carbon atoms, benzoyl and a benzoyl substituted in any position by at least one halogen, C₁ - C₃ alkyl, C₁ - C₃ alkoxy, nitro and trifluoromethyl groups; and R₂ is any nucleoside base analog.

2. The compound of Claim 1 wherein:
R₂ is a heterocyclic radical selected from: 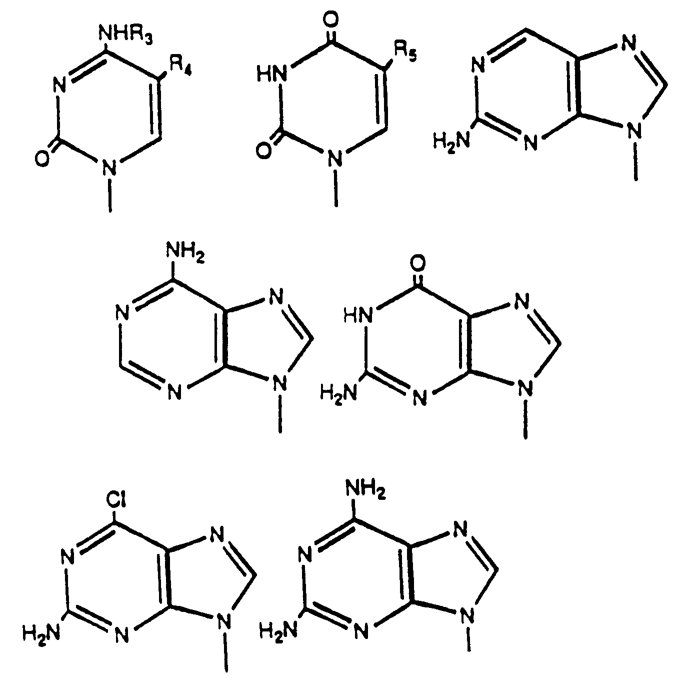 wherein:
R₃ and R₄ are independently selected from the group consisting of hydrogen and C₁ - C₃ alkyl; and
R₅ is selected from the group consisting of C₁ - C₃ alkyl and halogen.

3. The compound of Claim 2 which is 2-acetoxymethyl-4-(thymin-1'-yl)-1,3-dioxolane.

4. The compound of Claim 2 which is 2-hydroxymethyl-4-(thymin-1'-yl)-1,3-dioxolane.

5. The compound of Claim 2 which is 2-benzoyloxymethyl-4-(cytosin-1'-yl)-1,3 dioxolane.

6. The compound of Claim 2 which is trans-2-hydroxymethyl-4-(cytosin-1'-yl)-1,3-dioxolane.

7. The compound of Claim 2 which is 2-benzoyloxymethyl-4-(adenin-9'-yl)-1,3-dioxolane.

8. The compound of Claim 2 which is 2-hydroxymethyl-4-(adenin-9'-yl)-1,3-dioxolane.

9. The compound of Claim 2 which is 2-benzoyloxylmethyl-4-(2'-amino-6'-chloro-purin-9'-yl)-1,3-dioxolane.

10. The compound of Claim 2 which is 2-hydroxymethyl-4-(2'-amino-6'-chloro-purin-9'-yl)-1,3-dioxolane.

11. The compound of Claim 2 which is 2-hydroxymethyl-4-(2'-amino-purin-9'-yl)-1,3-dioxolane.

12. The compound of Claim 2 which is 2-hydroxymethyl-4-(2',6'-diamino-purin-9'-yl)-1,3-dioxolane.

13. The compound of claim 2 which is 2-hydroxymethyl-4-(guanin-9'-yl)-1,3-dioxolane.

14. A pharmaceutical composition for treating viral infections comprising a 1,3-dioxolane derivative as claimed in any of Claims 1 to 13 and a pharmaceutically acceptable carrier.

15. The use of a 1,3-dioxolane derivative as claimed in any of Claims 1 to 13 in the manufacture of a pharmaceutical composition for treating viral infections.

## Claims (Claims for the following Contracting State(s): ES)

1. A process for manufacture of a pharmaceutical composition comprising mixing a 1,3-dioxolane derivative of general formula (L), the geometric and optical isomers thereof, or mixtures of these isomers: 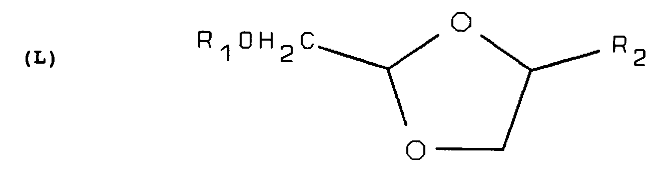 wherein R₁ is selected from the group consisting of hydrogen, an acyl group having 1 to 16 carbon atoms, benzoyl and a benzoyl substituted in any position by at least 1 halogen, C₁ - C₃ alkyl, C₁ - C₃ alkoxy, nitro and trifluoromethyl groups; and R₂ is any nucleoside base analogue,
with a pharmaceutically acceptable carrier.

2. A process according to Claims 1 wherein R₂ is a heterocylic radical selected from: 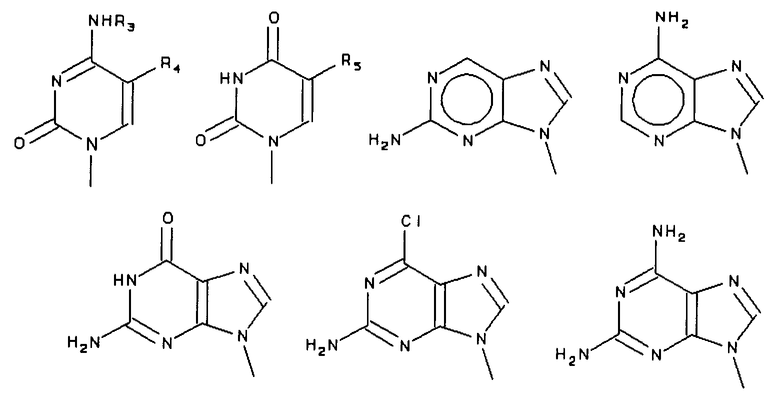 wherein R₃ and R₄ are independently selected from the group consisting of hydrogen and C₁ - C₃ alkyl; and R₅ is selected from the group consisting of C₁ - C₃ alkyl and halogen.

3. A process according to Claim 1 or 2 wherein the 1,3-dioxolane derivative of general formula (L) is selected from the following:-
2 - acetoxymethyl-4-(thymin-1'-yl)-1,3-dioxolane,
2 - hydroxymethyl-4-(thymin-1'-yl)-1,3-dioxolane,
2 - benzoyloxymethyl-4-(cytosin-1'-yl)-1',3-dioxolane
trans-2-hydroxymethyl-4-(cytosin-1'yl)-1,3-dioxolane,
2 - benzoloxymethyl-4-(adenin-9'-yl)-1,3-dioxolane,
2 - hydroxymethyl-4-(adenin-9'-yl)-1,3-dioxolane,
2 - benzoyloxylmethyl-4-(2'-amino-6'-chloro-purin-9'-yl)-1,3-dioxolane,
2 - hydroxymethyl-4-(2'-amino-6'-chloro-purin-9'-yl)-1,3-dioxolane,
2 - hydroxymethyl-4-(2'-amino-purin-9'-yl)-1,3-dioxolane,
2 - hydroxymethyl-4-(2',6'-diamino-purin-9'-yl)-1,3-dioxolane, and
2 - hydroxymethyl-4-(guanin-9'-yl)-1,3-dioxolane.

## Claims (Claims for the following Contracting State(s): GR)

1. A process for manufacture of a pharmaceutical composition comprising mixing a 1,3-dioxolane derivative of general formula (L), the geometric and optical isomers thereof, or mixtures of these isomers: 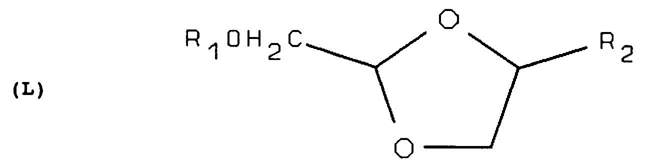 wherein R₁ is selected from the group consisting of hydrogen, an acyl group having 1 to 16 carbon atoms, benzoyl and a benzoyl substituted in any position by at least 1 halogen, C₁ - C₃ alkyl, C₁ - C₃ alkoxy, nitro and trifluoromethyl groups; and R₂ is any nucleoside base analogue,
with a pharmaceutically acceptable carrier.

2. A process according to Claims 1 wherein R₂ is a heterocylic radical selected from: 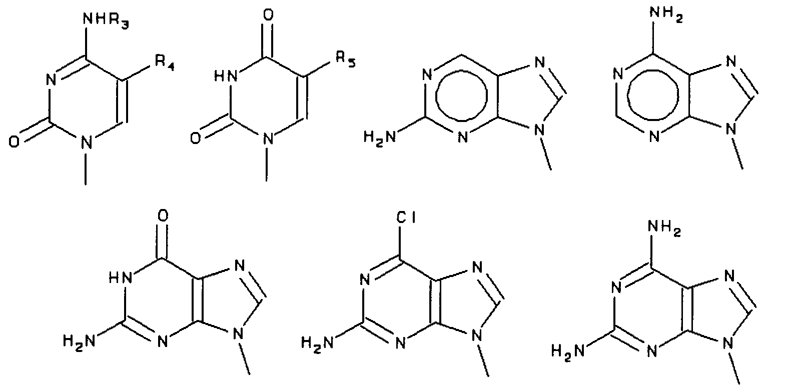 wherein R₃ and R₄ are independently selected from the group consisting of hydrogen and C₁ - C₃ alkyl; and R₅ is selected from the group consisting of C₁ - C₃ alkyl and halogen.

3. A process according to Claim 1 or 2 wherein the 1,3-dioxolane derivative of general formula (L) is selected from the following:-
2 - acetoxymethyl-4-(thymin-1'-yl)-1,3-dioxolane,
2 - hydroxymethyl-4-(thymin-1'-yl)-1,3-dioxolane,
2 - benzoyloxymethyl-4-(cytosin-1'-yl)-1,3-dioxolane
trans-2-hydroxymethyl-4-(cytosin-1'-yl)-1,3-dioxolane,
2 - benzoyloxymethyl-4-(adenin-9'-yl)-1,3-dioxolane,
2 - hydroxymethyl-4-(adenin-9'-yl)-1,3-dioxolane,
2 - benzoyloxymethyl-4-(2'-amino-6'-chloro-purin-9'-yl)-1,3-dioxolane,
2 - hydroxymethyl-4-(2'-amino-6'-chloro-purin-9'-yl)-1,3-dioxolane,
2 - hydroxymethyl-4-(2'-amino-purin-9'-yl)-1,3-dioxolane,
2 - hydroxymethyl-4-(2',6'-diamino-purin-9'-yl)-1,3-dioxolane, and
2 - hydroxymethyl-4-(guanin-9'-yl)-1,3-dioxolane.

4. Use of a 1,3-dioxolane derivative of general formula (L) according to any of Claims 1-3 in the manufacture of a medicament for treating viral infections.

## Patentansprüche (Patentansprüche für folgende(n) Vertragsstaat(en): AT, BE, CH, DE, FR, GB, IT, LI, LU, NL, SE)

1. 1,3-Dioxolanderivat der allgemeinen Formel (L), 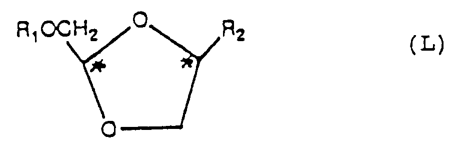 in der
R₁ aus der aus Wasserstoff, einer Acylgruppe mit 1 bis 16 Kohlenstoffatomen, Benzoyl und in beliebiger Position durch mindestens eine Halogen-, C₁₋₃-Alkyl-, C₁₋₃-Alkoxy-, Nitro- oder Trifluormethylgruppe substituiertem Benzoyl bestehenden Gruppe ausgewählt ist, und R₂ ein beliebiges Analogon einer Nukleosidbase darstellt, sowie
geometrische und optische Isomere desselben und Gemische dieser Isomere.

2. Verbindung gemäß Anspruch 1, in der R₂ einen aus 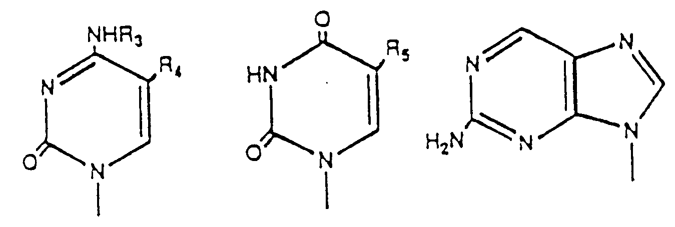 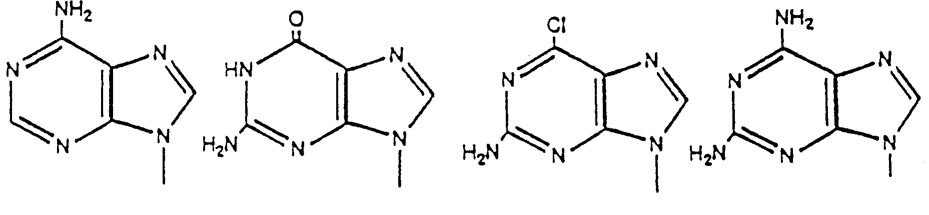 ausgewählten heterocyclischen Rest darstellt, wobei
R₃ und R₄ unabhängig voneinander aus der aus Wasserstoff und C₁₋₃-Alkyl bestehenden Gruppe und R₅ aus der aus C₁₋₃-Alkyl und Halogen bestehenden Gruppe ausgewählt ist.

3. Verbindung gemäß Anspruch 2, bei der es sich um 2-Acetoxymethyl-4-(thymin-1'-yl)-1,3-dioxolan handelt.

4. Verbindung gemäß Anspruch 2, bei der es sich um 2-Hydroxymethyl-4-(thymin-1'-yl)-1,3-dioxolan handelt.

5. Verbindung gemäß Anspruch 2, bei der es sich um 2-Benzoyloxymethyl-4-(cytosin-1'-yl)-1,3-dioxolan handelt.

6. Verbindung gemäß Anspruch 2, bei der es sich um trans-2-Hydroxymethyl-4-(cytosin-1'-yl)-1,3-dioxolan handelt.

7. Verbindung gemäß Anspruch 2, bei der es sich um 2-Benzoyloxymethyl-4-(adenin-9'-yl)-1,3-dioxolan handelt.

8. Verbindung gemäß Anspruch 2, bei der es sich um 2-Hydroxymethyl-4-(adenin-9'-yl)-1,3-dioxolan handelt.

9. Verbindung gemäß Anspruch 2, bei der es sich um 2-Benzoyloxymethyl-4-(2'-amino-6'-chlorpurin-9'-yl)-1,3-dioxolan handelt.

10. Verbindung gemäß Anspruch 2, bei der es sich um 2-Hydroxymethyl-4-(2'-amino-6'-chlorpurin-9'-yl)-1,3-dioxolan handelt.

11. Verbindung gemäß Anspruch 2, bei der es sich um 2-Hydroxymethyl-4-(2'-aminopurin-9'-yl)-1,3-dioxolan handelt.

12. Verbindung gemäß Anspruch 2, bei der es sich um 2-Hydroxymethyl-4-(2',6'-diaminopurin-9'-yl)-1,3-dioxolan handelt.

13. Verbindung gemäß Anspruch 2, bei der es sich um 2-Hydroxymethyl-4-(guanin-9'-yl)-1,3-dioxolan handelt.

14. Arzneimittel zur Behandlung viraler Infektionen, das ein 1,3-Dioxolanderivat gemäß einem der Anprüche 1 bis 13 und einen pharmazeutisch geeigneten Träger enthält.

15. Verwendung eines 1,3-Dioxolanderivates gemäß einem der Ansprüche 1 bis 13 zur Herstellung eines Arzneimittels zur Behandlung viraler Infektionen.

## Patentansprüche (Patentansprüche für folgende(n) Vertragsstaat(en): ES)

1. Verfahren zur Herstellung eines Arzneimittels, bei dem man ein 1,3-Dioxolanderivat der allgemeinen Formel (L), 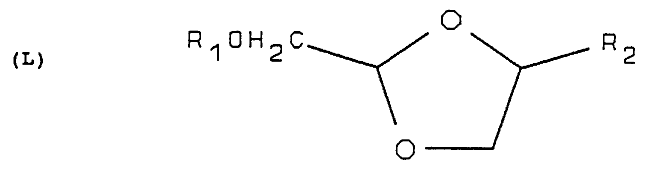 in der
R₁ aus der aus Wasserstoff, einer Acylgruppe mit 1 bis 16 Kohlenstoffatomen, Benzoyl und in beliebiger Position durch mindestens eine Halogen-, C₁₋₃-Alkyl-, C₁₋₃ Alkoxy-, Nitro- oder Trifluormethylgruppe substituiertem Benzoyl bestehenden Gruppe ausgewählt ist und R₂ ein beliebiges Analogon einer Nukleosidbase darstellt, sowie deren
geometrische und optische Isomere und Gemische dieser Isomere
mit einem pharmazeutisch geeigneten Träger mischt.

2. Verfahren gemäß Anspruch 1, wobei R₂ einen aus 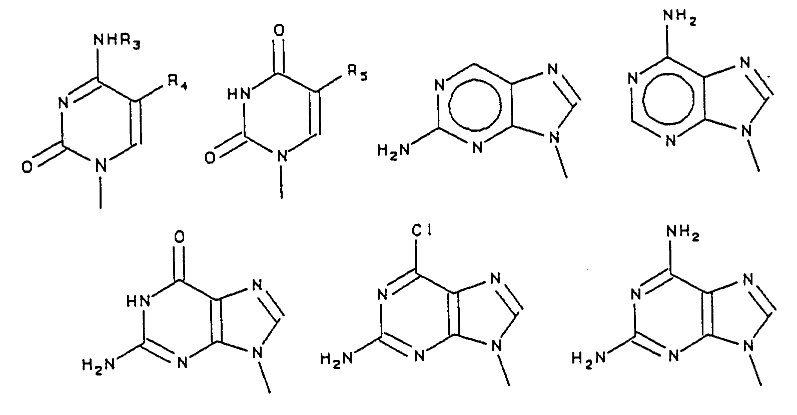 ausgewählten heterocyclischen Rest darstellt und
R₃ und R₄ unabhängig voneinander aus der aus Wasserstoff und C₁₋₃-Alkyl bestehenden Gruppe und R₅ aus der aus C₁₋₃-Alkyl und Halogen bestehenden Gruppe ausgewählt ist.

3. Verfahren gemäß Anspruch 1 oder 2, bei dem das 1,3-Dioxolanderivat der allgemeinen Formel (L) unter den folgenden gewählt wird:
- 2-Acetoxymethyl-4-(thymin-1'-yl)-1,3-dioxolan
- 2-Hydroxymethyl-4-(thymin-1'-yl)-1,3-dioxolan
- 2-Benzoyloxymethyl-4-(cytosin-1'-yl)-1,3-dioxolan
- trans-2-Hydroxymethyl-4-(cytosin-1'-yl)-1,3-dioxolan
- 2-Benzoyloxymethyl-4-(adenin-9'-yl)-1,3-dioxolan
- 2-Hydroxymethyl-4-(adenin-9'-yl)-1,3-dioxolan
- 2-Benzoyloxymethyl-4-(2'-amino-6'-chlorpurin-9'-yl)-1,3-dioxolan
- 2-Hydroxymethyl-4-(2'-amino-6'-chlorpurin-9'-yl)-1,3-dioxolan
- 2-Hydroxymethyl-4-(2'-aminopurin-9'-yl)-1,3-dioxolan
- 2-Hydroxymethyl-4-(2',6'-diaminopurin-9'-yl)-1,3-dioxolan
- 2-Hydroxymethyl-4-(guanin-9'-yl)-1,3-dioxolan

## Patentansprüche (Patentansprüche für folgende(n) Vertragsstaat(en): GR)

1. Verfahren zur Herstellung eines Arzneimittels, bei dem man ein 1,3-Dioxolanderivat der allgemeinen Formel (L), 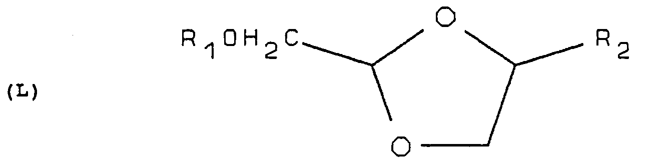 in der
R₁ aus der aus Wasserstoff, einer Acylgruppe mit 1 bis 16 Kohlenstoffatomen, Benzoyl und in beliebiger Position durch mindestens eine Halogen-, C₁₋₃-Alkyl-, C₁₋₃-Alkoxy-, Nitro- oder Trifluormethylgruppe substituiertem Benzoyl bestehenden Gruppe ausgewählt ist und R₂ ein beliebiges Analogon einer Nukleosidbase darstellt, sowie deren
geometrische und optische Isomere und Gemische dieser Isomere
mit einem pharmazeutisch geeigneten Träger mischt.

2. Verfahren gemäß Anspruch 1, wobei R₂ einen aus 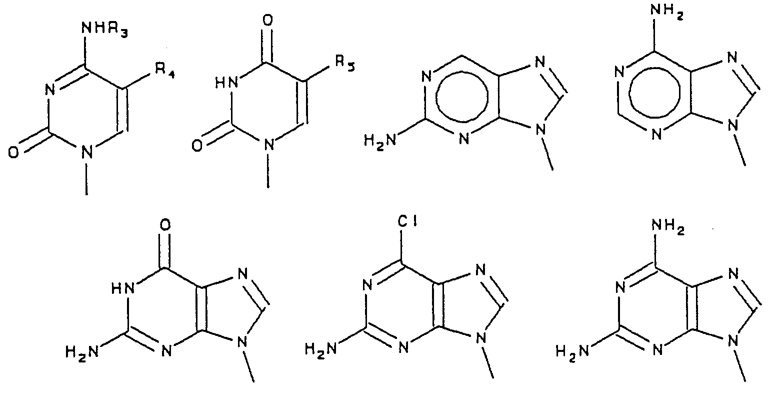 ausgewählten heterocyclischen Rest darstellt und
R₃ und R₄ unabhängig voneinander aus der aus Wasserstoff und C₁₋₃-Alkyl bestehenden Gruppe und R₅ aus der aus C₁₋₃-Alkyl und Halogen bestehenden Gruppe ausgewählt ist.

3. Verfahren gemaß Anspruch 1 oder 2, bei dem das 1,3-Dioxolanderivat der allgemeinen Formel (L) unter den folgenden gewählt wird:
- 2-Acetoxymethyl-4-(thymin-1'-yl)-1,3-dioxolan
- 2-Hydroxymethyl-4(thymin-1'-yl)-1,3-dioxolan
- 2-Benzoyloxymethyl-4-(cytosin-1'-yl)-1,3-dioxolan
- trans-2-Hydroxymethyl-4-(cytosin-1'-yl)-1,3-dioxolan
- 2-Benzoyloxymethyl-4-(adenin-9'-yl)-1,3-dioxolan
- 2-Hydroxymethyl-4-(adenin-9'-yl)-1,3-dioxolan
- 2-Benzoyloxymethyl4-(2'-amino-6'-chlorpurin-9'-yl)-1,3-dioxolan
- 2-Hydroxymethyl-4-(2'-amino-6'-chlorpurin-9'-yl)-1,3-dioxolan
- 2-Hydroxymethyl-4-(2'-aminopurin-9'-yl)-1,3-dioxolan
- 2-Hydroxymethyl-4-(2',6'-diaminopurin-9'-yl)-1,3-dioxolan
- 2-Hydroxymethyl-4-(guanin-9'-yl)-1,3-dioxolan

4. Verwendung eines 1,3-Dioxolanderivates der allgemeinen Formel (L) gemäß einem der Ansprüche 1 bis 3 zur Herstellung eines Arzneimittels für die Behandlung viraler Infektionen.

## Revendications (Revendications pour l'(les) Etat(s) contractant(s) suivant(s): AT, BE, CH, DE, FR, GB, IT, LI, LU, NL, SE)

1. Dérivé de 1,3-dioxalane de formule générale (L), isomères géométriques et optiques de celui-ci, et mélanges de ces isomères : 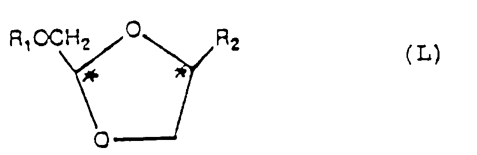 dans laquelle :
- R₁ est choisi au sein du groupe comprenant l'hydrogène, un groupe acyle comportant 1 à 16 atomes de carbone, les groupes benzoyle et benzoyle substitué en une position quelconque par au moins un atome d'halogéne, et par les groupes alkyle C₁-C₃, alcoxy C₁-C₃, nitro et trifluorométhyle; et
- R₂ est un analogue d'une base nucléoside quelconque.

2. Composé selon la revendication 1, dans lequel :
- R₂ est un radical hétérocyclique choisi parmi : 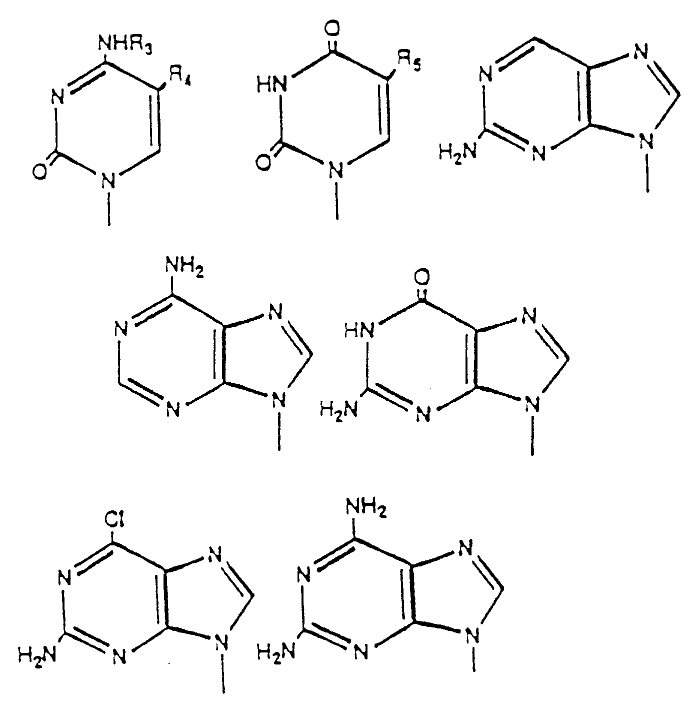 dans lequel :
- R₃ et R₄ sont choisis indépendamment au sein du groupe comprenant l'hydrogène et les groupes alkyle C₁-C₃; et
- R₅ est choisi parmi le groupe comprenant les groupes alkyle C₁-C₃ et un halogène.

3. Composé selon la revendication 2, qui est le 2-acétoxyméthyl-4-(thymin-1'-yl)-1,3-dioxalane.

4. Composé selon la revendication 2, qui est le 2-hydroxyméthyl-4-(thymin-1'-yl)-1,3-dioxalane.

5. Composé selon la revendication 2, qui est le 2-benzoyloxyméthyl-4-(cytosin-1'-yl)-1,3-dioxalane.

6. Composé selon la revendication 2, qui est le trans 2-hydroxyméthyl-4-(cytosin-1'-yl)-1,3-dioxalane.

7. Composé selon la revendication 2, qui est le 2-benzoyloxyméthyl-4-(adénin-9'-yl)-1,3-dioxalane.

8. Composé selon la revendication 2, qui est le 2-hydroxyméthyl-4-(adénin-9'-yl)-1,3-dioxalane.

9. Composé selon la revendication 2, qui est le 2-benzoyloxylméthyl-4-(2'-amino-6'-chloro-purin-9'-yl)-1,3-dioxalane.

10. Composé selon la revendication 2, qui est le 2-hydroxyméthyl-4-(2'-amino-6'-chloro-purin-9'-yl)-1,3-dioxalane.

11. Composé selon la revendication 2, qui est le 2-hydroxyméthyl-4-(2'-amino-purin-9'-yl)-1,3-dioxalane.

12. Composé selon la revendication 2, qui est le 2-hydroxyméthyl-4-(2',6'-diamino-purin-9'-yl)-1,3-dioxalane.

13. Composé selon la revendication 2, qui est le 2-hydroxyméthyl-4-(guanin-9'-yl)-1,3-dioxalane.

14. Composition pharmaceutique pour le traitement d'infections virales, comprenant un dérivé de 1,3-dioxalane selon l'une quelconque des revendications 1 à 13, et un véhicule pharmaceutiquement acceptable

15. Utilisation d'un dérivé de 1,3-dioxalane selon l'une quelconque des revendications 1 à 13, dans la fabrication d'une composition pharmaceutique pour le traitement d'infections virales.

## Revendications (Revendications pour l'(les) Etat(s) contractant(s) suivant(s): ES)

1. Procédé pour la fabrication d'une composition pharmaceutique, comprenant le mélange d'un dérivé de 1,3-dioxalane de formule générale (L), d'isomères géométriques et optiques de celui-ci, ou de mélanges de ces isomères : 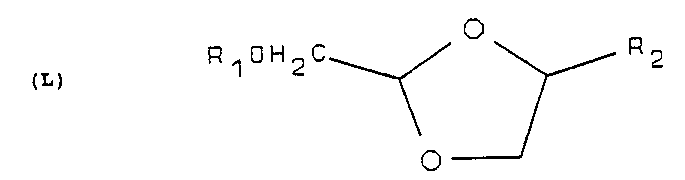 dans laquelle :
- R₁ est choisi au sein du groupe comprenant l'hydrogène, un groupe acyle comportant 1 à 16 atomes de carbone, les groupes benzoyle et benzoyle substitué en une position quelconque par au moins un atome d'halogène, et par les groupes alkyle C₁-C₃, alcoxy C₁-C₃, nitro et trifluorométhyle; et
- R₂ est un analogue d'une base nucléoside quelconque,
avec un véhicule pharmaceutiquement acceptable.

2. Procédé selon la revendication 1, dans lequel R₂ est un radical hétérocyclique choisi parmi : 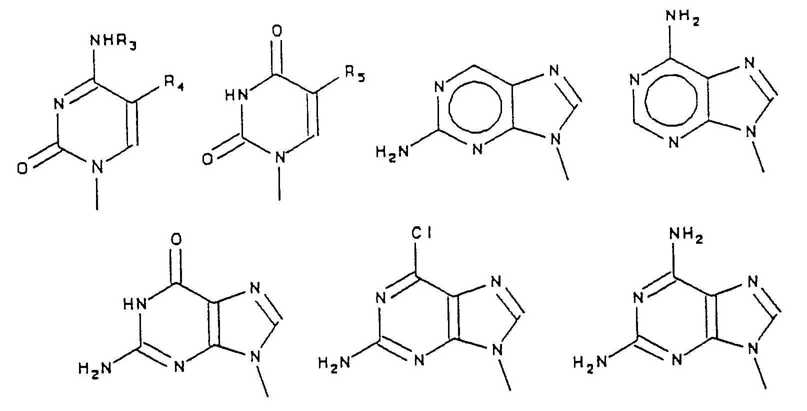 dans lequel :
- R₃ et R₄ sont choisis indépendamment au sein du groupe comprenant l'hydrogène et les groupes alkyle C₁-C₃; et R₅ est choisi parmi le groupe comprenant les groupes alkyle C₁-C₃ et un halogène.

3. Procédé selon la revendication 1, ou la revendication 2, dans lequel le dérivé de 1,3-dioxalane de formule générale (L) est choisi parmi les composés suivants :
- le 2-acétoxyméthyl-4-(thymin-1'-yl)-1,3-dioxalane,
- le 2-hydroxyméthyl-4-(thymin-1'-yl)-1,3-dioxalane,
- le 2-benzoyloxyméthyl-4-(cytosin-1'-yl)-1,3-dioxalane,
- le trans 2-hydroxyméthyl-4-(cytosin-1'-yl)-1,3-dioxalane,
- le 2-benzoyloxyméthyl-4-(adénin-9'-yl)-1,3-dioxalane,
- le 2-hydroxyméthyl-4-(adénin-9'-yl)-1,3-dioxalane.
- le 2-benzoyloxylméthyl-4-(2'-amino-6'-chloro-purin-9'-yl)-1,3-dioxalane,
- le 2-hydroxyméthyl-4-(2'-amino-6'-chloro-purin-9'-yl)-1,3-dioxalane,
- le 2-hydroxyméthyl-4-(2'-amino-purin-9'-yl)-1,3-dioxalane.
- le 2-hydroxyméthyl-4-(2',6'-diamino-purin-9'-yl)-1,3-dioxalane, et
- le 2-hydroxyméthyl-4-(guanin-9'-yl)-1,3-dioxalane.

## Revendications (Revendications pour l'(les) Etat(s) contractant(s) suivant(s): GR)

1. Procédé pour la fabrication d'une composition pharmaceutique, comprenant le mixage d'un dérivé de 1,3-dioxalane de formule générale (L), d'isomères géométriques et optiques de celui-ci, ou de mélanges de ces isomères : 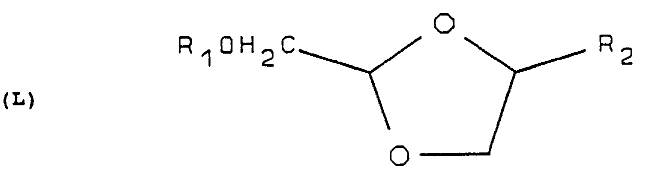 dans laquelle :
- R₁ est choisi au sein du groupe comprenant l'hydrogène, un groupe acyle comportant 1 à 16 atomes de carbone, les groupes benzoyle et benzoyle substitué en une position quelconque par au moins un atome d'halogène, et par les groupes alkyle C₁-C₃, alcoxy C₁-C₃, nitro et trifluorométhyle; et
- R₂ est un analogue d'une base nucléoside quelconque,
avec un véhicule pharmaceutiquement acceptable.

2. Procédé selon la revendication 1, dans lequel R₂ est un radical hétérocyclique choisi parmi : 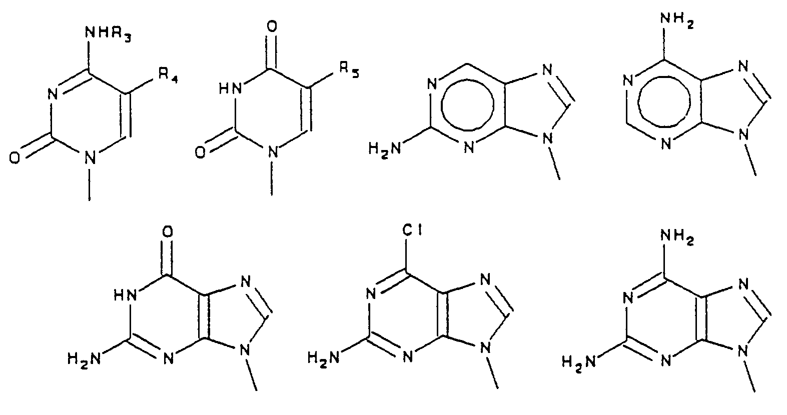 dans lequel :
- R₃ et R₄ sont choisis indépendamment au sein du groupe comprenant l'hydrogène et les groupes alkyle C₁-C₃; et R₅ est choisi parmi le groupe comprenant les groupes alkyle C₁-C₃ et un halogène.

3. Procédé selon la revendication 1, ou la revendication 2, dans lequel le dérivé de 1,3-dioxalane de formule générale (L) est choisi parmi les composés suivants :
- le 2-acétoxyméthyl-4-(thymin-1'-yl)-1,3-dioxalane,
- le 2-hydroxyméthyl-4-(thymin-1'-yl)-1,3-dioxalane,
- le 2-benzoyloxyméthyl-4-(cytosin-1'-yl)-1,3-dioxalane,
- le trans 2-hydroxyméthyl-4-(cytosin-1'-yl)-1,3-dioxalane,
- le 2-benzoyloxyméthyl-4-(adénin-9'-yl)-1,3-dioxalane,
- le 2-hydroxyméthyl-4-(adénin-9'-yl)-1,3-dioxalane.
- le 2-benzoyloxylméthyl-4-(2'-amino-6'-chloro-purin-9'-yl)-1,3-dioxalane,
- le 2-hydroxyméthyl-4-(2'-amino-6'-chloro-purin-9'-yl)-1,3-dioxalane,
- le 2-hydroxyméthyl-4-(2'-amino-purin-9'-yl)-1,3-dioxalane.
- le 2-hydroxyméthyl-4-(2',6'-diamino-purin-9'-yl)-1,3-dioxalane, et
- le 2-hydroxyméthyl-4-(guanin-9'-yl)-1,3-dioxalane.

4. Utilisation d'un dérivé de 1,3-dioxalane de formule générale (L) selon l'une quelconque des revendications 1 à 3, dans la fabrication d'un médicament pour le traitement d'infections virales.
